# EUROPEAN PATENT APPLICATION

(11) **EP 4 588 639 A1**
(43) Date of publication of application: **23.07.2025**
(21) Application number: 24152736.5
(22) Date of filing: 18.01.2024
(51) Int. Cl.: B28C 7/16, G01N 27/74, G01N 33/38, G01F 1/002, G01F 1/66, G01F 1/74

(54) **CONCRETE DUMPER**

(71) Applicant: NV Bekaert SA, 8550 Zwevegem (BE); Topp & Screed, 4410 Soleuvre (LU)
(72) Inventor: JIMÉNEZ, Cristián, 9840 De Pinte (BE); THOOFT, Hendrik, 9700 Oudenaarde (BE); PIÉRARD, Baudouin, 6860 Léglise (BE); NEUBERG, Bertrand, 6717 Attert (BE); DUHAUTPAS, Serge, 9089 Ettelbruck (LU); DROGUEST, Damien, 6740 Étalle (BE)
(74) Representative: Gevers Patents

(57) **Abstract**

The invention relates to a concrete dumper (1) equipped with an apparatus (2) arranged in a chute (3) of the dumper (1) for measuring and/or monitoring the distribution of a magnetic material presents in the concrete when the latter flows within the chute (3).

## Description

### Technical field

The invention relates to a concrete dumper.

### Prior art

In the field of building, it is known to use a "dumper" (also known as a "dumper truck") to carry bulk material on building sites. For a bulk material being concrete and/or similar flowable material, such a dumper will be referred to as a "concrete dumper" in this document. Such a dumper generally comprises a tray mounted on a wheeled frame for receiving and containing concrete and a chute fluidically coupled to the tray for distributing the concrete to a specific location of a building site. It may further comprise a space or a cabin mounted on the frame, sized to receive an operator and comprising a control unit configured for enabling the operator to control dumper movements and/or operations.

A concrete dumper is typically smaller than a concrete mixer (which is also known as a "truck mixer" in the framework of the present document) and is used to transport the concrete on a location to which such a mixer cannot go. In such case, a concrete dumper is easier and advantageous to use in comparison to concrete pump for instance.

For improving sustainability as well as stress and strain resistance of a concrete building, it is known to add steel fibers into the concrete for reinforcing it. The steel fibers are generally added to the other bulk materials constituting the concrete when the latter is still in the concrete mixer, so that there are mixed with these other bulk materials. It is however very important to control as regularly as possible the amount of steel fibers added to the concrete and/or the distribution of these steel fibers over the concrete volume in order to guarantee the concrete reinforcement quality.

In order to solve this problem, the publication EP2923184B1 provides an apparatus comprising a first device to determine a volume flow of a flow of a flowable substance comprising concrete and additional magnetic material (such as steel fibers), a second device to determine a signal caused by the additional magnetic material present in said flow, and tools to convert the signal measured by the second device in a signal per unit of volume of the flow of the flowable substance. These first and second devices are located at remote points X and Y both along the flow.

According to a main use of EP2923184B1, the devices are arranged to be removably placed at the lower extremity of the chute of a concrete mixer for automatically measuring and/or monitoring the distribution of steel fibers present in a flow of a steel fibers reinforced concrete during an unloading of the concrete by the concrete mixer. It is then advantageously possible to guarantee that the steel fibers are homogeneously distributed over the concrete flow, in particular prior to its transfer from the concrete mixer to a concrete dumper if the latter is needed to carry the concrete to a specific location of a building site.

This process can however be improved. Indeed, the devices need to be placed at the lower extremity of concrete mixer chute, then removed and cleaned with independent means between each flow of concrete, which takes times and energy. This is further exacerbated given that the apparatus involves measuring devices, notably said second device sensitive to magnetic material, and then needs to be manipulated and cleaned with high precautions, avoiding any impacts or metallic residue.

### Disclosure of the invention

An object of the present invention is to provide concrete carrying and distribution means that allows to improve the efficiency of steel fibers distribution measuring and/or monitoring in a reinforced concrete flow.

For this purpose, the present invention provides a concrete dumper comprising a movable body and a chute extending from the body and comprising a higher extremity and a lower extremity. The concrete dumper is configured to distribute a concrete to a location so that the concrete flows from the body to the higher extremity of the chute then, within the chute, from higher extremity to the lower extremity. A part of the chute remote from the lower extremity is equipped with an apparatus configured for measuring and/or monitoring the distribution of a magnetic material, for instance steel fibers used for reinforcing the concrete, presents in the concrete when the latter flows within said part of the chute.

The concrete dumper according to the invention allows to carry and to distribute a steel fibers reinforced concrete while measuring and/or monitoring more efficiently the steel fibers distribution in the concrete flow. Indeed, in place of considering a removable apparatus placed at the lower extremity of a concrete mixer chute, the invention provides an apparatus at least partially integrated in a higher part of a concrete dumper chute as said part of the chute equipped with the apparatus is remote from the chute lower extremity. This provides several advantages.

First, it is not needed anymore to remove the apparatus or its first and second devices between each concrete flow as these are integrated to a concrete carrying machine. This is possible because a concrete dumper is dedicated to carrying and distributing concrete on a site and remain on this site, while several different concrete mixers follow one another to bring concrete on the site. While it is not efficient and damaging to the apparatus to endow each mixer (mainly not on site) with such an apparatus, it is easy to endow the one or a few numbers of concrete dumpers dedicated to the site with such an apparatus.

Second, the position of all or part of the apparatus integrated in the chute is remote from the chute lower extremity to avoid possible collision and/or damage to the apparatus which has to be manipulated with precaution. This is easily possible because the apparatus does not need to be removed, otherwise, it would have been needed to place it at the lower extremity of the chute as in the prior art for making it possible to place and remove the apparatus or at least the first and second devices. Preferably, said part of the chute is closer to the chute higher extremity than to the chute lower extremity to limit furthermore the risk of damage to the apparatus.

Third, a steel fibers distribution measuring and/or monitoring is made at a later stage, in particular when the concrete dumper distributes the concrete and not when the concrete mixer provides the concrete to the concrete dumper. It allows then to guarantee the steel fibers distribution at the last step of concrete distribution, which may not be the case according to the prior art.

As mentioned above, in the framework of this document, a "concrete dumper" referred to a dumper provided for carrying bulk material being concrete and/or a similar flowable material such as mortar. The term "concrete" is used in this document generically. This term designates preferably a material comprising cement, water and bulk material aggregated with the cement. The concrete may also comprises admixture materials and/or additional magnetic material such as steel reinforcing fibers. The skilled person will understand that the invention may equivalently be implemented with similar flowable materials preferably including an additional magnetic material. Preferably, the "concrete" refers to a steel fibers reinforced concrete.

In the framework of this document, the use of the verb "comprise" or variants, does not exclude the presence of other elements than those mentioned. The use in this document of the terms "a", "an" or "the", to introduce an element does not exclude the presence of several of these elements. In this document, the terms "first", "second" and the like are used for distinguishing similar elements without inducing sequential or chronological order, unless otherwise indicated.

Preferably, the body of the concrete dumper comprises a fixed open tray mounted on a wheeled frame for receiving and containing concrete, the chute being fluidically coupled to the tray. The tray is fixed in the sense it is not able to move (in particular in rotation) relatively to the frame. It is open preferably on the tray top to allows the concrete to be distributed in the tray by a concrete mixer.

The concrete dumper body is movable via the wheels of the wheeled frame. As it is known by the skilled person, the dumper is typically endowed with a motor and/or an energy source for its movements and operation. It preferably includes a control unit for controlling movements and/or operations of the dumper such as a flowing of the concrete from the tray to the chute.

Preferably, the chute of the concrete dumper is open from the higher extremity to the lower extremity. This allows to monitor visually the concrete flow and to guarantee that potential part, such as a sensor, of the apparatus are not in contact with the concrete if needed. The chute then forms and/or comprises an open channel for the concrete.

According to an embodiment of the invention, said apparatus forms said part of the chute. In other word, said part of the chute is formed by at least part of the apparatus. Advantageously, this at least part of the apparatus is then integrated as part of the chute, and the concrete can flows through the apparatus as it flows within the whole chute. According to such embodiment, the apparatus comprises a channel portion forming a smooth and continuous extension of the other part of the chute. Such channel portion may enclose one or more sensors for measuring data used for determining said distribution of a magnetic material presents in the concrete.

According to an embodiment of the invention, the apparatus comprises a detector part including sensors configured for determining data related to said distribution of magnetic material and a data processing system to receive the data and to convert them into said distribution of magnetic material.

The detector part is preferably a one piece element. It is preferably the part of the apparatus forming said part of the chute as mentioned hereabove. It comprises said channel portion. The data processing system may be integrated in a single piece apparatus also comprising the detector part. It can be endowed with a screen or the like for showing information related to the measuring or the monitoring of said distribution. It may also be advantageous for the apparatus to be made up of two separated part: the detector part fully integrated to the chute and the data processing system that can be moved with an operator and that is configured for receiving (e.g. by wireless connection) said data from the detector part.

According to an embodiment of the invention, the apparatus, or more specifically at least its detector part, is fixed to other parts of the chute, the fixing being non-removable, notably in a normal use of the concrete dumper. It may be fixed by screwing or any other similar known technics. Preferably, following the preceding embodiment, its channel portion is maintained fixed to the other parts of the chute by a large number of bolts. Such fixing means is made so that it can be removed in specific circumstances, for instance for the maintenance of the apparatus or for replacing it in case of any malfunction. Advantageously, as the apparatus is non-removably fixed to the chute in a normal use of the dumper, its fixing can be improved with respect to the prior art removable placement of the apparatus at the lower extremity of a mixer chute. This leads to the effect that the relative movements that the detector part can undergo are reduced in a normal use of the dumper, then further reducing the risk of damage to the apparatus.

According to an embodiment of the invention, the apparatus, or more specifically at least its detector part, is directly fixed to the chute higher extremity. In this document, the term "directly fixed" is used as referring to a fixing with mechanical contact between two elements. As the apparatus (or at least said detector part) is at the level of the higher extremity, so close or at the level of the coupling of the chute to the body, it is then further protected against damages that could be induced by the chute movements. This is all the more important as the dumper chute generally moves laterally and vertically faster and further than a mixer chute. This fixing is preferably non-removable in a normal use of the dumper as mentioned above.

According to an embodiment of the invention, the concrete dumper comprises a sprayer overlooking said part of the chute. The sprayer is arranged to spray a cleaning liquid, preferably water, on the apparatus, more specifically at least its detector part. Advantageously, a sprayer integration around said part of the chute is made possible and useful as the detector part of the apparatus is itself integrated to the chute. It allows to clean at least the detector part of the apparatus automatically and efficiently, without additional cleaning means, after each concrete flow. This allows to avoid magnetic residues or other substances to remain on the detector part as it can affect the magnetic material distribution measuring.

According to an embodiment of the invention, the apparatus is directly electrically connected to an energy source of the body (or more generally of the dumper). As the apparatus is integrated to the chute, it allows to use directly the energy source of the dumper to supply the apparatus in energy, without a need for a specific energy source such as a battery used according to the prior art for a removable apparatus.

According to a particular embodiment of the invention, the apparatus comprises:
- sensors along said part of the chute to determine data related to:
   - a volume flow of concrete within said part of the chute, and
   - a signal caused by said magnetic material presents in the concrete;
- a data processing system to receive the data determined by the sensors and to convert them into a distribution of said magnetic material presents in the concrete when the latter flows within the said part of the chute.

The data may be transmitted by the sensors to the data processing system by a wireless connection. The data processing system may comprise a computer, a smartphone or the like, which comprises means for converting the data into said distribution in real time.

The signal is preferably proportional to the inductance and/or magnetic properties of the magnetic material. The apparatus may in particular be realized as described in the publication EP2923184B1, such realization being then hereby incorporated by reference. In particular, the data processing system may convert data received as a signal per unit of volume of a concrete flow which is then used to determine said distribution. This latter step can be implemented by proportion backwards of said signal per unit of volume.

According to an embodiment, the sensors comprise:
- an inductance sensor arranged in a channel portion of the apparatus forming said part of the chute; and/or
- an ultrasonic sensor arranged over said part of the chute.
This configuration is easy and compact to implement in a single detector part of the apparatus while it allows to protect the sensor arranged in the channel portion from damage.

The ultrasonic sensor may be placed above said part of the chute, and maintained fixed by a bridge extending over the channel portion from one side to an opposite side of the latter. The ultrasonic sensor is oriented toward the channel portion to sense the height and/or the volume of concrete under it (then under a defined cross-sectional area). As the concrete flow velocity can be determined by known technics such as such an ultrasonic sensor, by visible inspection and/or by measuring a signal caused by the concrete flow (e.g. including markers and sensor of the markers among the sensors), the data obtained allows the data processing system to determine the volume flow of concrete.

One or more inductance sensors are preferably arranged on opposed lateral sides of the channel portion. These sensors may generate a signal that is proportional to the inductance caused by the magnetic material presents in the concrete. The data processing system can associate the signal to an amount of magnetic material presents in the concrete flowing within said part of the chute (or in the defined cross-sectional area) in real time. The latter and the determined volume flow are then used by the data processing system to obtain said signal per unit of volume of a concrete flow.

### Brief description of the figures

Other features and advantages of the present invention will appear on reading the detailed descript that follows, for the understanding of which, it is referred to the attached drawings.

The list of these drawings is the following:
- Figure 1 illustrates a tridimensional view of a concrete dumper according to a preferred embodiment of the invention;
- Figure 2 illustrates an enlarged view of the chute of the concrete dumper that is illustrated in Figure 1.

The drawings are typically not scaled. Similar elements are generally assigned by similar references. In the framework of this document, identical or analogous elements may have the same references. Moreover, the presence of reference in the drawings cannot be considered to be limiting, comprising when these references are indicated in the claims.

### Detailed description of an embodiment of the invention

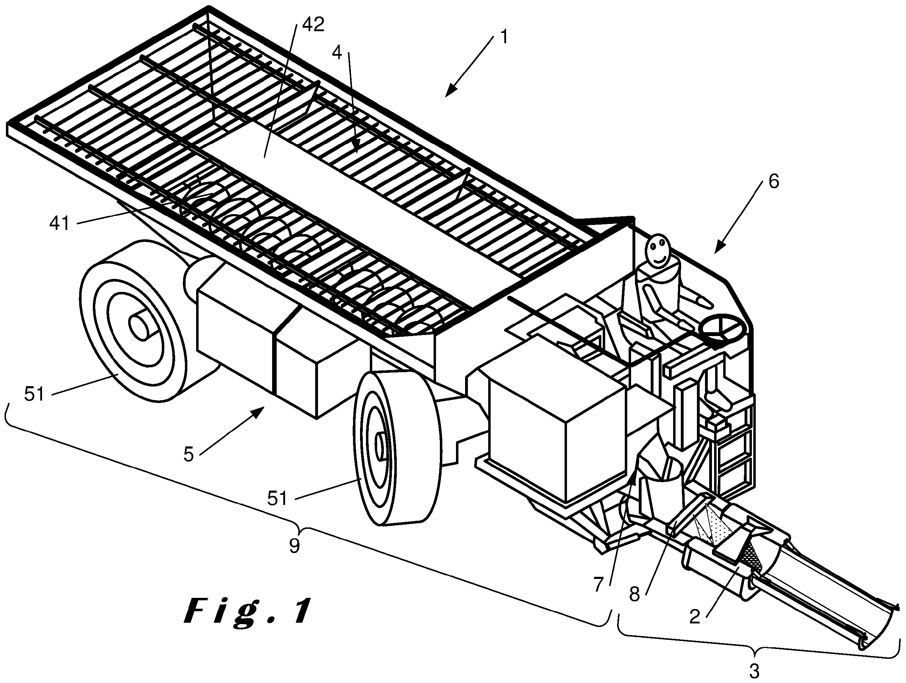

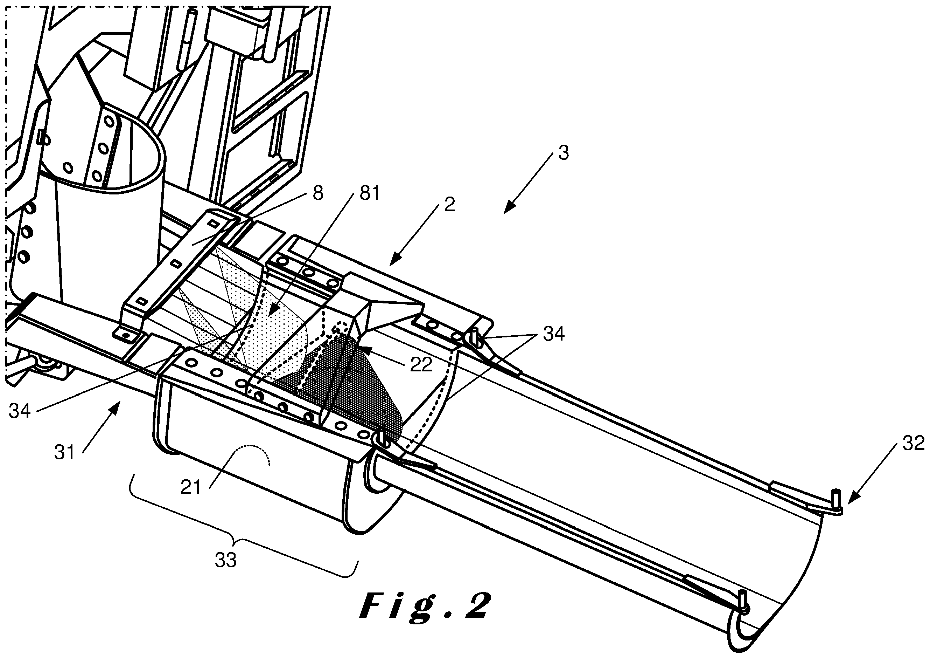

This part of this document presents a description of an embodiment of the invention with references to the drawings. The invention is however not limited by these references. The Figures 1 and 2 introduced above are in particular only schematic and not limiting in any way.

A concrete dumper 1 according to the invention is illustrated in Figure 1. It comprises a movable body 9 and a chute 3 extending from the body 9 that allows to distribute a concrete to a location on a building site. The dumper body 9 comprises a frame 5 mounted on wheels and supporting a fixed tray 4. As it is known, the body 9 also comprises a motor and/or an energy source for supplying the dumper components in energy for its movements and its operation.

The tray 4 is arranged to receive concrete, for instance from a mixer, through an opening that can be totally or partially closed by a grille or the like 42. The tray 4 may include an helicoidal blade able to rotate on itself for keeping the concrete in movement. The chute 3 is fluidically coupled to the tray 4 through a channel 7 endowed with a mechanism in order to allow or not and to control the concrete flow through the channel 7, to the chute 3.

The dumper body 9 also comprises a cabin 6 mounted on the frame 5 and sized to receive an operator as shown in Figure 1. The cabin 6 comprises a control unit of the dumper movements and operations that the operator can used for this purpose.

As illustrated in Figure 2, the chute 3 is open along its extension and comprises a higher extremity 31 and a lower extremity 32 that are defined so that the concrete flows from higher extremity 31 to the lower extremity 32 within the chute 3. The chute 3 further integrates an apparatus 2 configured for measuring and monitoring the distribution of steel fibers presents in the concrete when the latter flows within the chute 3.

The apparatus 2 forms a part of the chute 33 directly fixed, higher, to the higher extremity 31 of the chute, and lower, to the other part of the chute 3, the fixing being made by a collection of bolts and pinning 34 on both sides of the apparatus 2. The lower extremity 32 may comprise pinning means for attaching an extension to the chute 3.

The apparatus 2 comprises a channel portion forming said part of the chute 33 as a smooth extension of the other parts of the chute 3. The channel portion encloses one or more sensors 21, for instance inductance sensors, that are configured for determining data associated to a signal caused by the steel fibers passing in the part of the chute 33. Preferably, sensors 21 are arranged on both opposite sides of the channel portion 33 so that the concrete flows between these.

The apparatus 2 also comprises a ultrasonic sensor 22 supported by a bridge over the channel portion and allowing to determine data related to the concrete volume flow within said part of the chute 33. The data determined by the sensors 21, 22 are sent to a data processing system (not shown) to convert them in real time into a distribution of the steel fibers present in the concrete. The data processing system is integrated in a single piece apparatus 2 in this realization but it can also be a separate element exchanging data by wireless connection with the rest of the apparatus shown in Figure 2. The apparatus may be provided with an interface (not shown) for an operator to enter and/or receive information.

The concrete dumper 1 comprises a sprayer 8 mounted on another mechanical bridge over the higher extremity 31 so that it overlooks said part of the chute 33. The sprayer is arranged for spraying water 81 on the apparatus 2 in order to clean it between two flows of concrete.

In brief, the above described invention relates to a concrete dumper 1 that is equipped with an apparatus 2 arranged in a chute 3 of the dumper 1 for measuring and/or monitoring the distribution of a magnetic material presents in the concrete when the latter flows within the chute 3.

It will be obvious to the person skilled in the art that the invention is not limited to the embodiment illustrated and/or described above, but that its scope is more broadly defined by the claims that are hereinafter introduced.

## Claims

1. Concrete dumper (1) comprising :
- a movable body (9); and
- a chute (3) extending from the body (9) and comprising a higher extremity (31) and a lower extremity (32);
the concrete dumper (1) being configured to distribute a concrete to a location so that the concrete flows from the body (9) to the higher extremity (31) of the chute (3) then, within the chute (3), from higher extremity (31) to the lower extremity (32);
**characterized in that** a part of the chute (33) remote from the lower extremity (32) equipped with an apparatus (2) configured for measuring and/or monitoring the distribution of a magnetic material presents in the concrete when the latter flows within said part of the chute (33).

2. Concrete dumper (1) according to claim 1, wherein the apparatus (2) forms said part of the chute (33).

3. Concrete dumper (1) according to claim 2, wherein the apparatus (2) comprises a detector part forming said part of the chute (33) and including sensors (21, 22) configured for determining data (21, 22) related to said distribution of magnetic material.

4. Concrete dumper (1) according to claim 3, wherein said detector part of the apparatus (2) is fixed to other parts of the chute (3), the fixing being non-removable in a normal use of the concrete dumper (1).

5. Concrete dumper (1) according to claim 3 or 4, wherein the detector part of the apparatus (2) is directly fixed to the higher extremity (31) of the chute (3).

6. Concrete dumper (1) according to any one of claims 1 to 5, comprising a sprayer (8) overlooking said part of the chute (33) and arranged to spray a cleaning liquid (81) on the apparatus (2).

7. Concrete dumper (1) according to any one of claims 1 to 6, wherein the apparatus (2) is directly electrically connected to an energy source of the body (9).

8. Concrete dumper (1) according to any one of claims 1 to 7, wherein the chute (3) is open between the higher extremity (31) and the lower extremity (32).

9. Concrete dumper (1) according to any one of claims 1 to 8, wherein the apparatus (2) comprises:
- sensors (21, 22) along said part of the chute (33) to determine data related to:
• a volume flow of concrete within said part of the chute (33), and
• a signal caused by said magnetic material presents in the concrete;
- a data processing system to receive the data determined by the sensors (21, 22) and to convert them into a distribution of said magnetic material presents in the concrete when the latter flows within the said part of the chute (33).

10. Concrete dumper (1) according to claim 9, wherein the sensors (21, 22) comprise:
- an inductance sensor (21) arranged in a channel portion of the apparatus forming said part of the chute (33); and
- an ultrasonic sensor (22) arranged over said part of the chute (33).

11. Concrete dumper (1) according to any one of claims 1 to 10, wherein the body (9) comprises a fixed open tray (4) mounted on a wheeled frame (5) for receiving and containing concrete, the chute (3) being fluidically coupled to the tray (4).
